# EUROPÄISCHE PATENTSCHRIFT

(11) **EP 0 551 792 B1**
(45) Veröffentlichungstag und Bekanntmachung des Hinweises auf die Patenterteilung: **28.02.1996**
(21) Anmeldenummer: 92810944.6
(22) Anmeldetag: 02.12.1992
(51) Int. Cl.: A61F 2/38

(54) **Meniskusplattform zu einem künstlichen Kniegelenk**
Meniscus platform for artificial knee joint
Plate-forme ménisque pour articulation artificielle du genou

(30) Priorität: 14.01.1992 CH 91/92
(43) Veröffentlichungstag der Anmeldung: 21.07.1993
(73) Patentinhaber: Wehrli, Ueli, Dr., 3084 Bern-Wabern (CH)
(72) Erfinder: Wehrli, Ueli, Dr. Med., CH-3084 Wabern (CH); Moser, Walter, Dr., CH-3037 Herrenschwanden (CH)
(74) Vertreter: Trieblnig, Adolf

(56) Entgegenhaltungen:
- EP-A- 0 021 421
- EP-A- 0 183 670
- US-A- 4 224 697

## Beschreibung

Die Erfindung handelt von einem künstlichen Kniegelenk für ein Knie, dessen hinteres Kreuzband und Seitenbänder voll intakt sind, bestehend aus einer metallischen Plattform, welche auf ihrer Unterseite über Verankerungselemente mit der Tibia verbunden ist, und bestehend aus metallischen Femurkondylen und aus einem oder mehreren Gleitkörpern, welche Kräfte zwischen der metallischen Plattform und den metallischen Femurkondylen übertragen.

Die Problematik und Lösungsformen für künstliche Kniegelenke sind ausführlich in EP-A-0 021 421 (US-A-4 309 778) beschrieben. Die gezeigten Lösungsformen sollen den Bewegungsmechanismus des natürlichen Kniegelenks nachahmen und möglichst sicher in Bezug auf die Führung der beweglichen Elemente gestalten. Sie beanspruchen einen für die Sicherung der Führungsbewegung entsprechend grossen Raumanteil, um den eine Resektion im Knochengewebe des Tibiaknochens vorgenommen werden muss.

Im natürlichen intakten Kniegelenk können vereinfachend drei Komponenten der Relativbewegungen zwischen den Femur- und den Tibiakondylen unterschieden werden:
- Translatorische Anterior-Posterior-Bewegung (Gleiten)
- Abrollen der Femurkondylen auf den Tibiakondylen in Flexion des Knies
- Rotation der Tibia gegenüber dem Femur um eine Achse parallel zur Tibiaachse.

In wieweit diese Bewegungen nach Implantation einer Schlitten-Prothese möglich sind, hängt von der jeweiligen Konstruktion und der Intaktheit des Band- und Muskelapparats ab. Weniger geführte Prothesen erlauben grössere translatorische und rotatorische Bewegungen der Tibia relativ zum Femur. Solche weniger geführte "less constraint" Prothesen sind gekennzeichnet durch eine geringere Kongruenz der Femur- und Tibiakondylen und eine kleinere und die Bewegung weniger begrenzende interkondyläre Eminentia.

Bei der z.B. in der Patentschrift US 4,309,778 beschriebenen Konstruktion mit zwei getrennten tibialen Gleitkomponenten aus Polyethylen werden translatorische und rotatorische Bewegungen in der Ebene des Gelenkes durch Verschiebung der in nutenförmigen Schienen geführten tibialen Gleitkomponenten erzeugt. Diese Führungsschienen sind auf kreissegmentförmigen Bahnen angeordnet. Das zur Knieflexion erforderlich Gleiten der Femurkondylen auf den Tibiakondylen erfolgt zwischen zueinander weitgehend kongruenten Flächen, wobei der tragende Bereich mit zunehmender Rotation um die Tibiaachse und/oder zunehmender Translation quer zur Tibiaachse abnimmt. Geringere Kongruenz der Gleitflächen ist jedoch verbunden mit grösseren Flächenpressungen, kann zu lokaler Ueberbeanspruchung, zu erhöhtem Polyethylenabrieb und schneller Zerstörung der Tibiakondylen aus Polyethylen führen. Die in den Schienen getrennt geführten "Menisken" weisen ein geringfügiges seitliches Spiel auf, um sich durch diese begrenzte freie Beweglichkeit zu den Femurkondylen zentrieren zu können. Die Bewegung der Polyethylen-Komponenten in den Nuten kann durch Ablagerungen, durch Gewebezellen und durch Polyethylenabrieb behindert werden.

Aufgabe der vorliegenden Erfindung ist es, kongruente Femur- und Tibiakondylen-Paarungen zu erzeugen, grössere Verschiebungen und Rotationen zwischen Femur und Tibia zuzulassen und den Anteil des abzutrennenden Knochenmaterials an der Tibia möglichst gering zu halten, ohne zu stark vom Bewegungsablauf des natürlichen Kniegelenks abzuweichen. Diese Aufgabe wird mit den kennzeichnenden Merkmalen von Anspruch 1 gelöst.

Mit Hilfe von drei getrennt beweglichen Gleitkörpern werden die Freiheitsgrade für die Führung entkoppelt und definierte, kongruente Kontaktverhältnisse zwischen Femur und Tibia geschaffen. Dies hat den Vorteil, dass grossflächige Kontaktverhältnisse über den gesamten Bewegungsbereich und eine hinreichende Kniegelenkstabilität erreicht werden. Die geringe Bauhöhe auf der Tibiaseite erlaubt es, dass durch den geringen Resektionsanteil das hintere Kreuzband ohne Anheben der Gelenkebene zu den Seitenbändern erhalten bleiben kann und sich an der Wegbegrenzung für bewegliche Elemente ähnlich dem natürlichen Knie beteiligen kann und dass somit für die Führung der beweglichen Elemente untereinander weniger mechanische Anschläge notwendig sind. Die Begrenzung der Freiheitsgrade wird durch die aktiven Strukturen (Muskeln) und passiven Strukturen (Bänder) erreicht. Es entstehen geringere Druckspitzen an den Führungselementen, was sich günstig auf das Verschleissverhalten auswirkt. Die Reduktion der Kraftspitzen wirkt sich günstig auf die Primärverankerung im Knochengewebe aus und verhindert die Lockerung von eingewachsenen Prothesenteilen. Die abhängigen Unteransprüche 2 bis 5 beziehen sich auf vorteilhafte Weiterbildungen der Erfindung.

Im folgenden wird die Erfindung von einem Ausführungsbeispiel beschrieben. Es zeigen:
- Figur 1: Schematisch die Ansicht eines Kniegelenks mit indirekter Führung der Femurkondylen durch die Eminentia bezüglich Rotation um die Tibiaachse und bezüglich Verschiebung längs der Mittellinie der Eminentia;
- Figur 2: schematisch die Ansicht eines Schnitts in der Frontalebene für eine Anordnung gemäss Figur 1;
- Figur 3: schematisch die Ansicht eines Kniegelenks mit direkter Führung der Femurkondylen durch die Eminentia bezüglich Rotation um die Tibiaachse und bezüglich Verschiebung längs der Mittellinie der Eminentia; und
- Figur 4: schematisch die Ansicht eines Schnitts in der Frontalebene für eine Anordnung gemäss Figur 3.

In den Figuren ist ein künstliches Kniegelenk gezeigt, bei dem das hintere Kreuzband und die Seitenbänder als erhalten vorausgesetzt sind. Eine metallische Plattform 1 ist an der Tibia 2 verankert und besitzt senkrecht zur Tibiaachse eine ebene Fläche 4, auf welcher drei Gleitkörper gleitbar angeordnet sind, um im Rahmen der ihnen unter dem Zug der Bänder verbliebenen Freiheitsgrade die metallische Plattform 1 relativ zu den metallisch ausgeführten Femurkondylen 10, 11 zu führen. Dabei bildet ein mittlerer Gleitkörper eine um einen Zapfen 5 drehbare Eminentia 6, welche die Femurkondylen 10, 11 direkt oder indirekt bezüglich Drehung um die Tibiaachse führt und welche längs ihrer Mittellinie 7, die in unverdrehter Mittellage in sagittaler Richtung verläuft, eine direkte oder indirekte Längsführung für die Femurkondylen 10, 11 bildet, während die Flexionsbewegung an doppelt gekrümmten Femurkondylen 10, 11 von den beiden äusseren Gleitkörpern 14, 15 geführt ist. Die Position der Gleitkörper zu den Femurkondylen und zueinander ist durch die Kongruenz, d.h. der sich ineinander zentrierenden Gleitflächen der Kondylenpaarung gegeben.

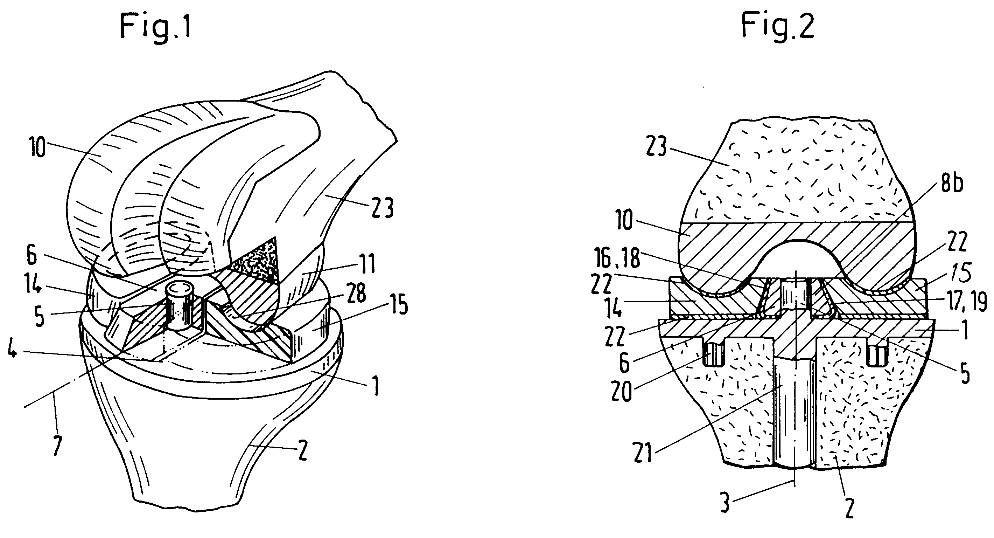

Figur 1 und 2 zeigen eine indirekte Führung der metallischen Femurkondylen 10, 11 durch die auf der ebenen Fläche 4 drehbar gelagerte Eminentia 6, wobei in der Mitte der metallischen Plattform 1 ein Zapfen 5 in Richtung der Tibiaachse 3 vorsteht, der in eine Bohrung der Eminentia 6 eingreift und der Schubkräfte in dieser ebenen Fläche 4 aufnehmen kann. Die Eminentia 6 ist als ein parallel zu ihrer Mittellinie verlaufender Wulst 8b ausgebildet, der zur Mittellinie 7 parallele Seitenflächen 16, 17 aufweist, an denen die äusseren Gleitkörper 14, 15 mit Gegenflächen 18, 19 anliegen. Die metallischen Femurkondylen 10, 11 weisen doppelt gekrümmte Flächen auf und halten über doppelt gekrümmten Gegenflächen 28 die äusseren Gleitkörper 14, 15 während der Flexion auf konstantem Abstand, der so bemessen ist, dass diese an den Seitenflächen 16, 17 der Eminentia 6 anliegen und geführt sind. Die äusseren Gleitkörper 14, 15 können auf diese Weise mit Planflächen an der Eminentia 6 und an der ebenen Fläche 4 geführt werden. Die planen Führungsflächen der Gleitkörper gewährleisten auch bei Gewebewucherungen einen störungsfreien Betrieb, während die doppelt gekrümmten Flächen der Femurkondylen 10, 11 die beispielsweise Ausschnitte aus einem Torus sind in unverändert grossen Gegenflächen 28 laufen. Die Lageabweichungen, die sich aus Fertigungstoleranzen bei Kongruenz d.h. bei optimaler Passung der doppelt gekrümmten Laufflächen für die äusseren Gleitkörper ergeben, können mit einem Spiel von 0,1 bis 0,3 mm der planen Seitenflächen 16, 17 der Eminentia 6 zwischen den Gegenflächen 18, 19 kompensiert werden. Das Gleiche gilt für Lageabweichungen zwischen den Femurkondylen 11, 12 wegen elastischer Deformationen auf der Femurseite.

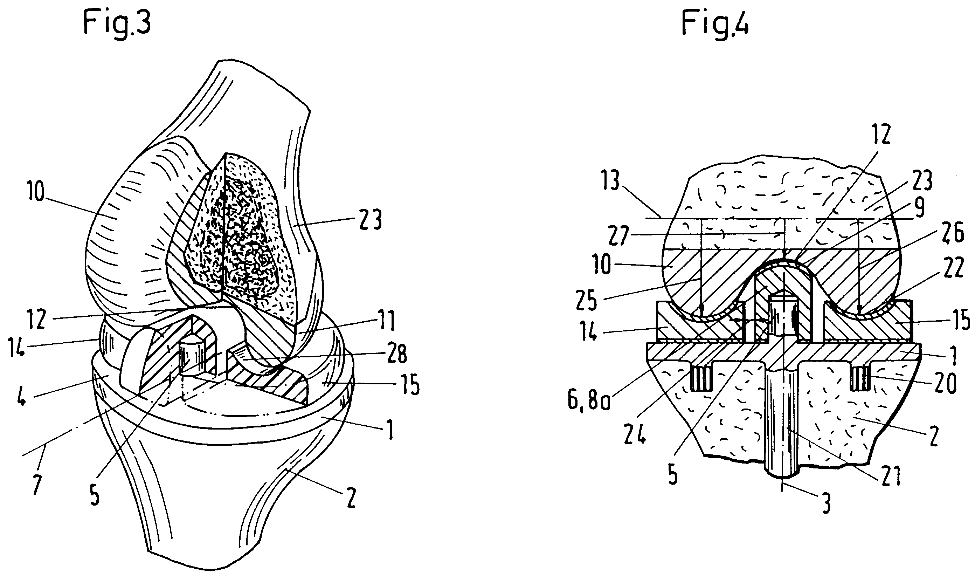

Figur 3 und 4 zeigen eine direkte Führung der metallischen Femurkondylen 10, 11 durch die auf der ebenen Fläche 4 drehbar gelagerte Eminentia 6, wobei in der Mitte der metallischen Plattform 1 ein Zapfen 5 in Richtung der Tibiaachse 3 vorsteht, der in eine Bohrung der Eminentia 6 eingreift und der Schubkräfte in dieser ebenen Fläche 4 aufnehmen kann. Die Eminentia ist als ein parallel zu ihrer Mittellinie verlaufender Wulst 8a ausgebildet, wobei die Kontur durch Verschieben einer gekrümmten Erzeugenden 9 parallel zu Mittellinie 7 erzeugt wird. Der Wulst 8a greift in eine Führungsrinne 12 zwischen den metallischen Femurkondylen 10, 11 ein, wobei in jedem Flexionswinkel die gekrümmte Erzeugende 9 für die Führungsrinne 12 die gleiche Drehachse 13 wie die Erzeugenden für die Femurkondylen aufweist. Die Differenz der Kondylenradien 25, 26 zum Radius 27 der Erzeugenden 9 bleibt für alle Flexionswinkel konstant. Ein Seitwärtskippen der metallischen Femurkondylen 10, 11 wird durch die äusseren Gleitkörper 14, 15 verhindert, die mit ihren doppelt gekrümmten Gegenflächen 28 durch die doppelt gekrümmten Flächen der metallischen Femurkondylen 10, 11 in der ebenen Fläche 4 geführt sind und einen Abstand 24 zur Eminentia aufweisen.

Dieser Abstand 24 reicht bei weitem aus, um die Lageabweichungen, die sich aus Fertigungstoleranzen und/oder elastischer Deformation bei Kongruenz der doppelt gekrümmten Laufflächen zwischen Femurkondylen 10, 11 und äusseren Gleitkörpern 14, 15 ergeben, aufzufangen. Erst bei einer Lockerung der Bänder und Vergrösserung des Abstandes zwischen Femur 23 und Tibia 2 in Richtung der Tibiaachse 3 kann sich der Abstand 24 soweit verringern, dass eine grobe Führung zwischen Eminentia und äusseren Gleitkörpern 14, 15 stattfinden kann. Auch hier bestehen kaum Störungsmöglichkeiten durch Gewebewucherungen. Die Gegenflächen 28 bleiben praktisch ganzflächig im Kontakt mit den Femurkondylen 10, 11, die äusseren Gleitkörper laufen auf Planflächen und die Führungsrinne 12 kann auf Grund ihrer offenen Form nicht durch Gewebewucherungen blockiert werden.

Bezüglich der Werkstoffe sind metallische Femurkondylen 10, 11 und eine metallische Plattform 1 vorgesehen, welche sich mit Verankerungselementen 20 in Form von Drehsicherungszapfen und mit einem Verankerungselement 21 in Form eines zentralen Verankerungsstiftes in der Tibia 2 abstützt. Die Eminentia 6 und die äusseren Gleitkörper 14, 15 können dabei wie in Figur 3 für die direkte Führung aus Kunststoff bestehen. Ebenso ist es möglich, die Eminentia 6 wie in Figur 1 für die indirekte Führung aus Metall und die äusseren Gleitkörper 14, 15 aus Kunststoff herzustellen. Die Figuren 2 und 4 zeigen Eminentia 6 und die äusseren Gleitkörper 14, 15 aus Metall, wobei deren Gleitflächen Oberflächenschichten 22 aufweisen, die zu einer verschleissarmen Paarung mit ihren metallischen Gegenflächen führen.

Die grossflächige Berührung zwischen Femurkondylen 10, 11 den äusseren Gleitkörpern 14, 15, der metallischen Plattform 1 und der Eminentia 6 erlaubt es für Eminentia und Gleitkörper neben Kunststoff auch Werkstoffe mit höheren E-Modulen, mit erhöhter Abriebbeständigkeit und mit erhöhter Festigkeit zu verwenden. Mit höherfesten Werkstoffen kann die Bauhöhe für Eminentia 6 und äussere Gleitkörper 14, 15 verringert werden und um das gleiche Mass eine geringere Resektion an der Tibia vorgenommen werden.

Die Figuren zeigen keine Patella-Prothesen, da diese in dem eingangs aufgeführten Stand der Technik enthalten sind.

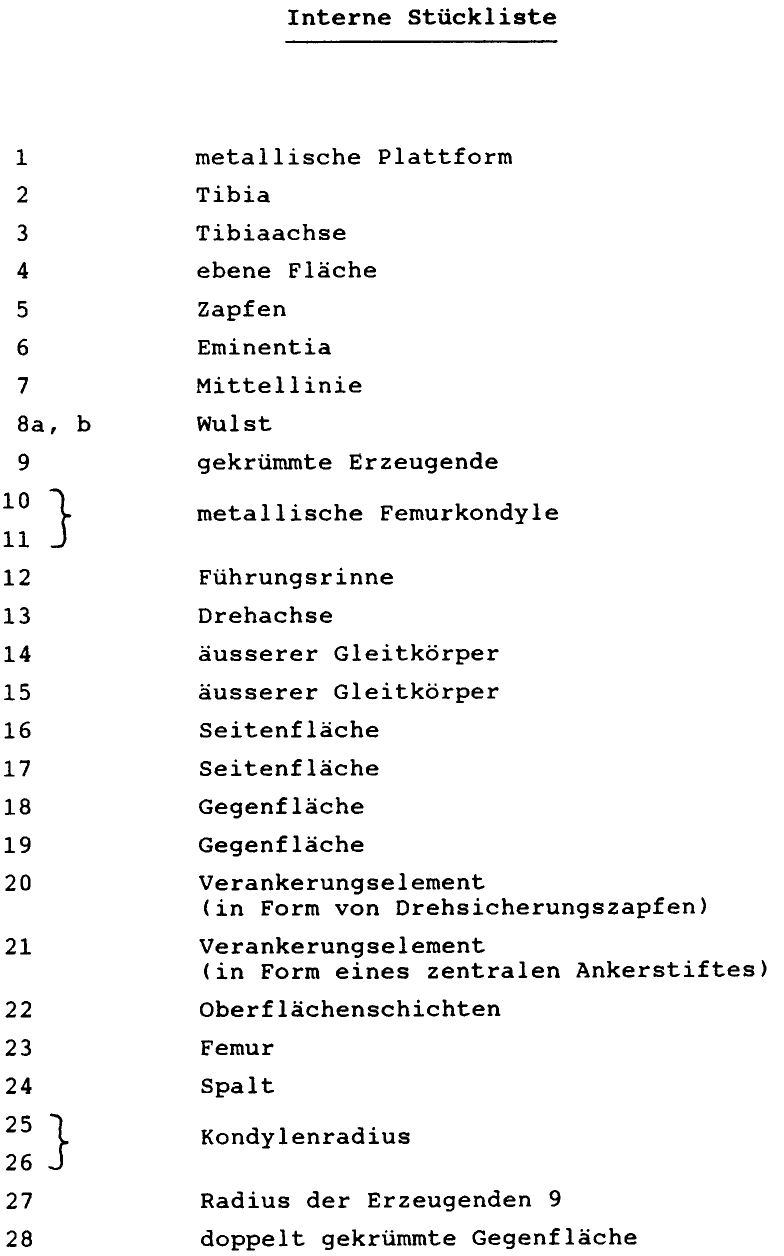

## Patentansprüche

1. Künstliches Kniegelenk für ein Knie, dessen hinteres Kreuzband und Seitenbänder voll intakt sind, bestehend aus einer metallischen Plattform (1), welche auf ihrer Unterseite über Verankerungselemente (20, 21) mit der Tibia (2) verbunden ist, und bestehend aus metallischen Femurkondylen (10, 11) und aus einem oder mehreren Gleitkörpern, welche Kräfte zwischen der metallischen Plattform (1) und den metallischen Femurkondylen (10, 11) übertragen, **dadurch gekennzeichnet,** dass drei von der Femurseite getrennte Gleitkörper auf einer ebenen Fläche (4) der metallischen Plattform (1) quer zur Tibiaachse (3) gleitbar angeordnet sind, wobei ein mittlerer Gleitkörper als eine um einen Zapfen (5) drehbare Eminentia (6) ausgebildet ist, welche die metallischen Femurkondylen (10, 11) direkt oder indirekt bezüglich Drehung um die Tibiaachse (3) führt und welche längs ihrer Mittellinie (7), die in unverdrehter Mittellage in sagittaler Richtung verläuft, eine direkte oder indirekte Längsführung für die Femurkondylen (10, 11) bildet, während die Flexionsbewegung an doppelt gekrümmten Femurkondylen (10, 11) von den beiden äusseren Gleitkörpern (14, 15) geführt ist.

2. Künstliches Kniegelenk nach Anspruch 1, dadurch gekennzeichnet, dass die direkte Führung der Femurkondylen (10, 11) bezüglich Drehung und längs der Mittellinie (7) der Eminentia (6) durch einen parallel zur Mittellinie verlaufenden Wulst (8a) mit einer gekrümmten Erzeugenden (9) vorgenommen ist, der in eine Führungsrinne (12) zwischen den Femurkondylen (10, 11) eingreift, wobei in jedem Flexionswinkel die gekrümmte Erzeugende (9) für die Führungsrinne (12) die gleiche Drehachse (13) wie die Erzeugenden für die Femurkondylen aufweist.

3. Künstliches Kniegelenk nach Anspruch 1, dadurch gekennzeichnet, dass die indirekte Führung der Femurkondylen (10, 11) bezüglich Drehung und längs der Mittellinie der Eminentia (6) durch einen parallel zur Mittellinie (7) verlaufenden Wulst (8a) vorgenommen ist, der zur Mittellinie (7) parallele Seitenflächen (16, 17) aufweist, an denen die äusseren Gleitkörper (14, 15) mit Gegenflächen (18, 19) unter der Zentrierwirkung der fest zueinander beabstandeten metallischen Femurkondylen (10, 11) anliegen, solange die äusseren Gleitkörper (14, 15) zwischen der metallischen Plattform (1) und/oder den Femurkondylen (10, 11) verspannt sind.

4. Künstliches Kniegelenk nach einem der Ansprüche 1 bis 3, dadurch gekennzeichnet, dass Eminentia (6) und/oder äussere Gleitkörper (14, 15) aus einem körperverträglichen Kunststoff bestehen.

5. Künstliches Kniegelenk nach einem der Ansprüche 1 bis 3, dass Eminentia (6) und äussere Gleitkörper (14, 15) aus Metall bestehen und mindestens an ihren Gleitflächen Oberflächenschichten (22) aufweisen, die zu einer verschleissarmen Paarung mit ihren metallischen Gegenflächen führen.

## Claims

1. An artificial knee joint for a knee, in which the rear cruciate ligament and the collateral ligaments are completely intact, consisting of a metallic platform (1), which on its under side is connected via attachment members (20, 21) to the tibia (2), and consisting of metallic femur condyles (10, 11) and of one or several slide members, which transmit forces between the metallic platform (1) and the metallic femur condyles (10, 11),
**characterised in that** three slide members separated from the femur side are slidably disposed on a plane face (4) of the metallic platform (1) at right angles to the tibia axis (3), with a central slide member being constructed as an eminentia (6) rotatable around a pin (5), which directly or indirectly guides the metallic femur condyles (10, 11) with respect to rotation around the tibia axis (3) and which along its median line (7), which extends in the sagittal direction in the non-rotated central position, forms a direct or indirect longitudinal guide for the femur condyles (10, 11), whereas the flexion movement at doubly curved femur condyles (10, 11) is guided by the two outer slide members (14, 15).

2. An artificial knee joint according to Claim 1,
**characterised in that** the direct guidance of the femur condyles (10, 11) with respect to rotation and along the median line (7) of the eminentia (6) is performed by a bead (8a), extending parallel to the median line, having a curved generatrix (9), which engages in a guide channel (12) between the femur condyles (10, 11), with the curved generatrix (9) for the guide channel (12) having the same axis of rotation (13) as the generatrices for the femur condyles in each flexion angle.

3. An artificial knee joint according to Claim 1,
**characterised in that** the indirect guidance of the femur condyles (10, 11) with respect to rotation and along the median line of the eminentia (6) is performed by a bead (8a) extending parallel to the median line (7), which comprises lateral faces (16, 17) parallel to the median line (7), against which the outer slide members (14, 15) abut with counter faces (18, 19) under the centring action of the metallic femur condyles (10, 11) having a fixed distance with respect to one another, as long as the outer slide members (14, 15) are braced between the metallic platform (1) and/or the femur condyles (10, 11).

4. An artificial knee joint according to one of Claims 1 to 3,
**characterised in that** eminentia (6) and/or outer slide members (14, 15) are made from a plastic which can be tolerated by the body.

5. An artificial knee joint according to one of Claims 1 to 3,
**characterised in that** eminentia (6) and outer slide members (14, 15) are made of metal and at least on their slide faces have surface layers (22) resulting in a wear-resistant combination with their metallic counter-surfaces.

## Revendications

1. Articulation artificielle du genou pour un genou dont le ligament croisé postérieur et les ligaments latéraux sont parfaitement intacts, constituée d'une plate-forme métallique (1) dont le dessous est relié au tibia (2) par l'intermédiaire d'éléments d'ancrage (20, 21), et constituée de condyles fémoraux métalliques (10, 11) et d'un ou plusieurs corps glissants qui transmettent des forces entre la plate-forme métallique (1) et les condyles fémoraux métalliques (10, 11), caractérisée en ce que trois corps glissants séparés du côté fémoral sont disposés sur une surface plane (4) de la plate-forme métallique (1) avec possibilité de glissement transversalement à l'axe du tibia (3), un corps glissant médian étant conçu sous la forme d'une éminence (6) qui peut pivoter autour d'un tourillon (5), qui guide les condyles fémoraux métalliques (10, 11) directement ou indirectement en rotation autour de l'axe du tibia et qui forme, le long de son axe médian (7), qui en position médiane non pivotée s'étend dans la direction sagittale, un guidage longitudinal direct ou indirect pour les condyles fémoraux (10, 11) tandis que le mouvement de flexion est guidé par les deux corps glissants extérieurs (14, 15) au niveau des condyles fémoraux à double courbure (10, 11).

2. Articulation artificielle du genou selon la revendication 1, caractérisée en ce que le guidage direct des condyles fémoraux (10, 11) en rotation et le long de l'axe médian (7) de l'éminence (6) est assuré par un bourrelet (8a) qui est parallèle à l'axe médian et qui présente une génératrice incurvée (9) pénétrant dans une échancrure de guidage (12) située entre les condyles fémoraux (10, 11), la génératrice incurvée (9) correspondant à l'échancrure de guidage (12) présentant, dans chaque angle de flexion, le même axe de rotation (13) que les génératrices correspondant aux condyles fémoraux.

3. Articulation artificielle du genou selon la revendication 1, caractérisée en ce que le guidage indirect des condyles fémoraux (10, 11) en rotation et le long de l'axe médian de l'éminence (6) est assuré par un bourrelet (8a) qui est parallèle à l'axe médian (7) et qui présente des surfaces latérales (16, 17) qui sont parallèles à l'axe médian (7) et contre lesquelles les corps glissants extérieurs (14, 15) sont appliqués par l'intermédiaire de surfaces antagonistes (18, 19) sous l'effet de centrage des condyles fémoraux métalliques (10, 11) à écartement mutuel fixe, aussi longtemps que les corps glissants extérieurs (14, 15) sont serrés entre la plate-forme métallique (1) et/ou les condyles fémoraux (10, 11).

4. Articulation artificielle du genou selon l'une des revendications 1 à 3, caractérisée en ce que l'éminence (6) et/ou les corps glissants extérieurs (14, 15) sont réalisés dans un matériau biocompatible.

5. Articulation artificielle du genou selon l'une des revendications 1 à 3, caractérisée en ce que l'éminence (6) et les corps glissants extérieurs (14, 15) sont réalisés en métal et comportent, au moins sur leurs surfaces de glissement, des couches superficielles (22) qui créent un appariement à faible usure avec leurs surfaces antagonistes métalliques.
